# FASCICULE DE BREVET EUROPEEN

(11) **EP 2 359 800 B1**
(45) Date de publication et mention de la délivrance du brevet: **12.09.2018**
(21) Numéro de dépôt: 10196191.0
(22) Date de dépôt: 21.12.2010
(51) Int. Cl.: A61K 8/31, A61K 8/34, A61K 8/60, A61Q 5/12

(54) **Composition de traitement cosmetique des fibres keratiniques a base d'alcane(s) lineraire(s) volatil(s), d'alcool(s) gras en C8-30 et de mono- ou polyglycoside(s)**
Zusammensetzung zur kosmetischen Behandlung von Keratinfasern, bestehend aus flüchtigem/en linearem/en Alkan/en, C8-C30-Fettalkohol/en und Mono- oder Polyglykosid(en)
Composition for cosmetic treatment of keratin fibres comprising volatile linear alkanes, C8-30 fatty alcohols and mono- or polyglycosides

(30) Priorité: 23.12.2009 FR 0959490
(43) Date de publication de la demande: 24.08.2011
(73) Titulaire: L'Oréal, 75008 Paris (FR)
(72) Inventeur: Degoul, Marie-Cécile, 75009 Paris (FR); Konate, Nadia, 92110 Clichy (FR)
(74) Mandataire: Dossmann, Gérard

(56) Documents cités:
- WO-A1-99/13844
- DE-A1-102008 012 457
- US-A1- 2008 269 352

## Description

La présente invention concerne une composition cosmétique comprenant au moins un alcane linéaire volatil, au moins un tensioactif amphotère ou zwittérionique, au moins un alcool gras en C₈₋₃₀ et au moins un mono- ou polyglycoside en un rapport pondéral alcool(s) gras/[mono- et/ou polyglycoside(s)] strictement supérieur à 2,5, son utilisation pour le traitement des fibres kératiniques, comme les cheveux, et un procédé de traitement cosmétique desdites matières kératiniques mettant en oeuvre ladite composition.

Dans le domaine du traitement capillaire, l'utilisation de solvants volatils est connue dans des produits capillaires de soins et de brillance. Ils sont généralement utilisés pour différentes raisons. Ils permettent notamment de modifier le rendu sensoriel d'un produit capillaire en lui conférant une texture légère et non collante dans la main. Ils peuvent également lui conférer un caractère glissant qui facilite la répartition du produit sur les cheveux et en particulier sur des cheveux secs. DE 10 2008 012457 se rapporte à l'utilisation des alcanes linéaires volatils dans les préparations pour les soins capillaires. Ces solvants volatils qui sont généralement des esters gras liquides, des huiles hydrocarbonées de type isododécane ou isohexadécane, et/ou des huiles siliconées, peuvent notamment conduire à des problèmes de toucher gras, de manque de brillance, et de cheveux raidis et durs.

Il subsiste donc un besoin de remplacer ces solvants volatils pour éviter les inconvénients cités ci-dessus.

La demanderesse a maintenant découvert de façon inattendue et surprenante, qu'une composition cosmétique comprenant au moins un alcane linéaire volatil, au moins un tensioactif amphotère ou zwittérionique, au moins un alcool gras en C₈₋₃₀ et au moins un mono-ou polyglycoside en un rapport pondéral alcool(s) gras/[ mono- et/ou polyglycoside(s)] strictement supérieur à 2,5, permettait de surmonter les inconvénients cités ci-dessus et d'améliorer de manière significative les propriétés cosmétiques des cheveux telles que le lissage, la tonicité, le démêlage, la légèreté et la souplesse.

En particulier, lors de l'application de la composition et après rinçage, on obtient des cheveux plus lisses et plus souples.

De plus, cette composition conduit notamment à des cheveux humides plus faciles à démêler, plus légers et plus toniques, et à des cheveux séchés plus souples et plus lisses au toucher.

Ainsi, l'invention a pour objet une composition cosmétique comprenant, dans un milieu cosmétiquement acceptable, un ou plusieurs alcanes linéaires volatils, un ou plusieurs tensioactifs amphotères ou zwittérioniques, un ou plusieurs alcools gras en C₈₋₃₀ et un ou plusieurs mono- ou polyglycosides en un rapport pondéral alcool(s) gras/[mono- et/ou polyglycoside(s)] strictement supérieur à 2,5.

Elle a encore pour objet l'utilisation de ladite composition pour le traitement cosmétique des matières kératiniques, de préférence des fibres kératiniques comme les cheveux.

Un autre objet de l'invention est un procédé de traitement cosmétique des matières kératiniques, de préférence des fibres kératiniques comme les cheveux, comprenant l'application de ladite composition sur lesdites matières kératiniques.

La composition cosmétique selon l'invention comprend, dans un milieu cosmétiquement acceptable :
- un ou plusieurs alcanes linéaires volatils,
- un ou plusieurs tensioactifs amphotères ou zwittérioniques,
- un ou plusieurs alcools gras en C₈₋₃₀, et
- un ou plusieurs mono- ou polyglycosides,
en un rapport pondéral alcool(s) gras/[mono- et/ou polyglycoside(s)] strictement supérieur à 2,5.

Ledit rapport pondéral va de préférence de 2,6 à 50, plus particulièrement de 3 à 40, mieux encore de 3 à 25 et encore mieux de 5 à 25.

Par « un ou plusieurs alcane(s) linéaire(s) volatil(s) », on entend indifféremment « une ou plusieurs huile alcane(s) linéaire(s) volatile(s) ».

Un alcane linéaire volatil convenant à l'invention est liquide à température ambiante (environ 25 °C) et sous pression atmosphérique (101 325 Pa ou 760 mm Hg).

Par « alcane linéaire volatil » convenant à l'invention, on entend un alcane linéaire cosmétique, susceptible de s'évaporer au contact de la peau en moins d'une heure, à température ambiante (25°C) et sous pression atmosphérique (101 325 Pa), liquide à température ambiante, ayant notamment une vitesse d'évaporation allant de 0,01 à 15 mg/cm²/min, à température ambiante (25°C) et sous pression atmosphérique (101 325 Pa).

De préférence, les alcanes linéaires volatils convenant à l'invention présentent une vitesse d'évaporation allant de 0,01 à 3,5 mg/cm²/min, mieux de 0,01 à 1,5 mg/cm²/min, à température ambiante (25°C) et sous pression atmosphérique (101 325 Pa).

De façon plus préférée, les alcanes linéaires volatils convenant à l'invention présentent une vitesse d'évaporation allant de 0,01 à 0,8 mg/cm²/min, préférentiellement de 0,01 à 0,3 mg/cm²/min, et encore plus préférentiellement de 0,01 à 0,12 mg/cm²/min, à température ambiante (25°C) et sous pression atmosphérique (101 325 Pa).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %), 15 g de solvant hydrocarboné volatil.

On laisse le solvant hydrocarboné volatil s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, les alcanes linéaires volatils convenant à l'invention ont une pression de vapeur (appelée également pression de vapeur saturante) non nulle, à température ambiante, en particulier une pression de vapeur allant de 0,3 Pa à 6000 Pa.

De façon préférée, les alcanes linéaires volatils convenant à l'invention ont une pression de vapeur allant de 0,3 à 2000 Pa, mieux de 0,3 à 1000 Pa, à température ambiante (25°C).

De façon plus préférée, les alcanes linéaires volatils convenant à l'invention ont une pression de vapeur allant de 0,4 à 600 Pa, préférentiellement de 1 à 200 Pa, et encore plus préférentiellement de 3 à 60 Pa, à température ambiante (25°C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, les alcanes linéaires volatils convenant dans l'invention peuvent être les alcanes linéaires comportant de 7 à 15 atomes de carbone, de préférence de 8 à 14 atomes de carbone et mieux de 9 à 14 atomes de carbone.

De façon plus préférée, les alcanes linéaires volatils convenant dans l'invention peuvent être les alcanes linéaires comportant de 10 à 14 atomes de carbone, et encore plus préférentiellement de 11 à 14 atomes de carbone.

Un alcane linéaire volatil convenant à l'invention peut être avantageusement d'origine végétale.

De préférence, l'alcane linéaire volatil ou le mélange d'alcanes linéaires volatils présent dans la composition selon l'invention comprend au moins un isotope ¹⁴C du carbone (carbone 14). En particulier, l'isotope ¹⁴C peut être présent en un rapport en nombre d'isotopes ¹⁴C/¹²C (ou ratio ¹⁴C/¹²C) supérieur ou égal à 1.10⁻¹⁶, de préférence supérieur ou égal à 1.10⁻¹⁵, de préférence encore supérieur ou égal 7,5.10⁻¹⁴, et mieux supérieur ou égal 1,5.10⁻¹³. De préférence, le ratio ¹⁴C / ¹²C va de 6.10⁻¹³ à 1,2.10⁻¹².

La quantité d'isotopes ¹⁴C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets WO 2007/068371 et WO2008/155059. Ces alcanes sont obtenus à partir d'alcools gras, eux-mêmes obtenus à partir d'huile de coprah ou de palme.

A titre d'exemples d'alcanes linéaires volatils convenant à l'invention, on peut citer le n- heptane (C7), le n-octane (C8), le n-nonane (C9), le n-décane (C10), le n-undécane (C11), le n-dodécane (C12), le n-tridécane (C13), le n-tétradécane (C14), le n-pentadécane (C15), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode de réalisation préféré, on peut citer les mélanges de n-undécane (C11) et de n-tridécane (C13) obtenus aux exemples 1 et 2 de la demande WO2008/155059.

On peut également citer le n-dodécane (C12) et le n-tétradécane (C14) vendus respectivement sous les références PARAFOL 12-97 et PARAFOL 14-97 par la société Sasol, ainsi que leurs mélanges.

Un mode de réalisation consiste à utiliser un seul alcane linéaire volatil.

Alternativement, on peut utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C10/C11, C11/C12, ou C12/C13.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et le mélange C11/C13 pour un nombre de carbone n impair.

Selon un mode de réalisation préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C11/C13 ou un mélange d'alcanes linéaires volatils C12/C14.

D'autres mélanges associant plus de deux alcanes linéaires volatils selon l'invention, tels que, par exemple, un mélange d'au moins trois alcanes linéaires volatils comportant de 7 à 15 atomes de carbone distincts et différant entre eux d'un nombre de carbone d'au moins 1, peuvent être utilisés dans l'invention.

Dans le cas des mélanges de deux alcanes linéaires volatils, lesdits deux alcanes linéaires volatils représentent de préférence plus de 95% et mieux plus de 99% en poids du mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cₙ avec n allant de 7 à 15
- de 10 à 50% en poids, de préférence de 20 à 45% en poids, de préférence de 24 à 40% en poids, d'alcane linéaire volatil en Cₙ₊ₓ avec x supérieur ou égal à 1, de préférence x=1 ou x=2, avec n+x compris entre 8 et 14,
par rapport au poids total des alcanes dans ledit mélange.

En particulier, ledit mélange d'alcanes linéaires volatils peut contenir en outre :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0,1% en poids d'hydrocarbures insaturés,
lesdits pourcentages étant exprimés par rapport au poids total du mélange.

Plus particulièrement, les alcanes linéaires volatils convenant dans l'invention peuvent être mis en oeuvre sous la forme d'un mélange n-undécane/n-tridécane.

En particulier, on utilisera un mélange d'alcanes linéaires volatils comprenant :
- de 55 à 80% en poids, de préférence de 60 à 75% en poids d'alcane linéaire volatil en C11 (n-undécane) et
- de 20 à 45% en poids, de préférence de 24 à 40% en poids d'alcane linéaire volatil en C13 (n-tridécane),
par rapport au poids total des alcanes dans ledit mélange.

Selon un mode de réalisation particulier, le mélange d'alcanes est un mélange n-undécane/n-tridécane. En particulier, un tel mélange peut être obtenu selon l'exemple 1 ou l'exemple 2 de la demande WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane.

Le ou les alcanes linéaires volatils sont utilisés de préférence en une quantité allant de 0,01 % à 30 % en poids, en particulier de 0,1 à 20% en poids, et plus particulièrement de 0,1 à 15% en poids, par rapport au poids total de la composition.

Le ou les alcool(s) gras en C₈₋₃₀ utilisé(s) dans l'invention sont de préférence des alcools gras linéaires ou ramifiés, saturés ou insaturés, en C₈₋₃₀, mieux en C₁₂-C₂₆. A titre d'exemples d'alcools gras pouvant être utilisés dans la présente invention, on peut notamment citer les alcools laurylique, myristylique, cétylique, stéarylique, cétéarylique, oléique, arachidylique et béhénylique, l'hexyldécanol, le 2-octyldodécanol, et leurs mélanges. De préférence, l'alcool gras utilisé dans l'invention est choisi parmi les alcools myristylique, cétylique, stéarylique et cétéarylique et leurs mélanges.

Le ou les alcools gras en C₈₋₃₀ peuvent être utilisés en une quantité allant de 0,5 à 20 % en poids, de préférence de 1 à 15% en poids, et plus particulièrement de 2 à 12% en poids, par rapport au poids total de la composition.

Les mono- ou polyglycosides sont des molécules obtenues à partir de la condensation d'un ou de plusieurs oses et de molécules non-glucidiques.

Les mono- ou polyglycosides utilisables dans l'invention sont bien connus et peuvent être plus particulièrement représentés par la formule générale suivante :

R₁O-(R₂O)ₜ(G)ᵥ (I)

dans laquelle :
- R₁ représente un groupe alkyle et/ou alcényle, linéaire ou ramifié, comportant environ de 8 à 24 atomes de carbone, un groupe alkylphényle dont le groupe alkyle linéaire ou ramifié comporte de 8 à 24 atomes de carbone,
- R₂ représente un groupe alkylène comportant environ de 2 à 4 atomes de carbone,
- G représente un motif sucre comportant de 5 à 6 atomes de carbone,
- t désigne une valeur allant de 0 à 10, de préférence 0 à 4, de préférence 0 à 4 et
- v désigne une valeur allant de 1 à 15.

Des mono- ou polyglycosides préférés dans la présente invention sont des (alkyl en C₈₋₁₈)(mono- ou polyglycosides) et sont des composés de formule (I) dans laquelle :
- R₁ désigne plus particulièrement un groupe alkyle saturé ou insaturé, linéaire ou ramifié comportant de 8 à 18 atomes de carbone,
- t désigne une valeur allant de 0 à 3 et plus particulièrement encore égale à 0,
- G peut désigner le glucose, le fructose ou le galactose, de préférence le glucose.

Le degré de polymérisation, i.e. la valeur de v dans la formule (I), peut aller de 1 à 15, de préférence de 1 à 4. Le degré moyen de polymérisation est plus particulièrement compris entre 1 et 2 et encore plus préférentiellement de 1,1 à 1,5.

Les liaisons glycosidiques entre les motifs sucre sont de type 1-6 ou 1-4, et de préférence 1-4.

Des exemples de composés de formule (I) sont notamment le caprylylglucoside, le décylglucoside (ou caprylglucoside), le laurylglucoside, le cétéarylglucoside, le cocoglucoside et leurs mélanges. Ces composés préférés de formule (I) peuvent être les produits vendus par la société COGNIS sous les dénominations PLANTAREN® (600 CS/U, 1200 et 2000) ou PLANTACARE® (818, 1200 et 2000). On peut également utiliser les produits vendus par la société SEPPIC sous les dénominations TRITON CG 110 (ou ORAMIX CG 110) et TRITON CG 312 (ou ORAMIX® NS 10), les produits vendus par la société B.A.S.F. sous la dénomination LUTENSOL GD 70 ou encore ceux vendus par la société CHEM Y sous la dénomination AG10 LK.

On peut également utiliser par exemple, l'(alkyl en C8/C16)polyglucoside-1,4 en solution aqueuse à 53% commercialisé par COGNIS sous la référence PLANTACARE® 818 UP.

Le ou les mono- ou polyglycosides peuvent être utilisés en une quantité allant de 0,1 à 20 % en poids, de préférence de 0,2 à 10% en poids, et plus particulièrement de 0,2 à 5% en poids, par rapport au poids total de la composition.

Le milieu cosmétiquement acceptable est constitué de préférence d'eau ou d'un mélange d'eau et d'au moins un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

La composition selon l'invention comprend en outre au moins un tensioactif amphotère ou zwitterionique.

Les tensioactifs amphotères utilisables dans la présente invention peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les (alkyl en C₈₋₃₀)bétaïnes, les sulfobétaïnes, les (alkyl en C₈₋₃₀)amido(alkyl en C₂₋₈)bétaïnes ou les (alkyl en C₈₋₃₀)amido(alkyl en C₂₋₈)sulfobétaïnes, et leurs mélanges.

Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (II) et (III) :

Rₐ-CONHCH₂CH₂-N(R_{b})(R_{c})(CH₂COO⁻) (II)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(B') (III)

dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylampho-dipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL® C2M concentré.

Parmi les tensioactifs amphotères ou zwittérioniques cités ci-dessus, on utilise de préférence les (alkyl en C₈₋₃₀)bétaïnes, les (alkyl en C₈₋₃₀)amido(alkyl en C₂₋₈)bétaïnes et leurs mélanges, et mieux encore l'olivamidopropylbétaïne.

Lorsqu'ils sont présents, la quantité du ou des tensioactifs amphotères ou zwittérioniques est de préférence comprise dans l'intervalle allant de 0,01 à 10 % en poids, en particulier de 0,1 à 8%, et plus particulièrement de 0,2 à 5 % en poids par rapport au poids total de la composition.

La composition cosmétique selon l'invention peut comprendre en outre un ou plusieurs composés naturels ou d'origine naturelle, tels que des actifs et extraits naturels comme des jus de fruits, des huiles essentielles et des extraits de plantes ; des huiles et des beurres d'origine végétale.

Par « composé naturel », on entend un produit que l'on obtient de la terre ou du sol, ou à partir de végétaux ou d'animaux, via éventuellement un ou des processus physiques comme, par exemple, un broyage, un raffinage, une distillation, une purification ou une filtration.

Par « composé d'origine naturelle », on entend un composé naturel ayant subi un ou plusieurs traitements chimiques n'affectant pas les qualités essentielles de ce composé et/ou un composé comprenant majoritairement des composés naturels.

La composition selon l'invention peut également comprendre en outre un ou plusieurs additifs classiques bien connus dans la technique, tels que des polysaccharides différents des polyglycosides, et particulièrement de ceux utilisés dans l'invention tels que définis ci-dessus, des tensio-actifs anioniques, cationiques ou non ioniques, des polymères anioniques, cationiques, non-ioniques, ou amphotères, des polyols, des protéines, des vitamines, des agents réducteurs, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des agents nacrants, des propulseurs, et des épaississants minéraux, ou leurs mélanges.

L'homme de métier veillera à choisir les éventuels additifs et leur quantité de manière à ce qu'ils ne nuisent pas aux propriétés des compositions cosmétiques selon l'invention.

Ces additifs sont généralement présents dans la composition selon l'invention en une quantité allant de 0 à 20 % en poids par rapport au poids total de la composition.

Les compositions cosmétiques selon l'invention peuvent se présenter sous la forme d'une lotion plus ou moins épaissie, d'une crème, d'un gel ou d'une émulsion.

Un autre objet de l'invention est l'utilisation de la composition cosmétique telle que décrite ci-dessus pour le traitement cosmétique des matières kératiniques, de préférence des fibres kératiniques comme les cheveux.

L'invention est également relative à un procédé de traitement cosmétique des matières kératiniques, de préférence des fibres kératiniques comme les cheveux, qui consiste à appliquer une quantité efficace d'une composition cosmétique telle que décrite ci-dessus, sur lesdites matières, à éventuellement rincer après un éventuel temps de pause.

Lorsque l'on applique la composition selon l'invention sous forme d'une lotion ou d'une crème, on la laisse éventuellement pauser sur les cheveux pendant environ 1/2 min. à 5 minutes, puis on rince éventuellement à l'eau.

Les exemples suivants sont donnés à titre illustratif de la présente invention.

Dans les exemples suivants, toutes les quantités sont indiquées en pour cent en poids par rapport au poids total de la composition, sauf indication contraire.

### EXEMPLES

### Exemple 1

On a préparé les compositions suivantes à partir des ingrédients indiqués dans le tableau ci-dessous.

| Composition | 1 | 2 | 3 | 4 | 5 |
|---|---|---|---|---|---|
| Mélange d'undécane et de tridécane⁽¹⁾ | 3 | 3 | 3 | - | 6 |
| Mélange de n-dodécane et de n-tétradécane⁽²⁾ | - | - | - | 3 | - |
| Alcool cétéarylique (C16/C18 50/50)⁽³⁾ | 3 | 4 | - | 3 | 6 |
| Alcool cétylique⁽⁴⁾ | - | - | 6 | - | - |
| Mélange (80/20 en poids) d'alcool cétéarylique et de cétéarylglucoside⁽⁵⁾ | 2 | - | 6 | 3 | 6 |
| Mélange (65/35 en poids) d'alcool cétéarylique et de cocoglucoside⁽⁶⁾ | - | 2 | - | - | - |
| Olivamidopropylbétaïne⁽⁷⁾ | 1 | 1 | - | 1 | 1 |
| Citrus aurantium Dulcis (orange) juice⁽⁸⁾ | 8,5 | 8,5 | 8,5 | 8,5 | 8,5 |
| Beurre de karité | 2 | 2 | 2 | 2 | 2 |
| Conservateur | 0,5 | 0,5 | 0,5 | 0,5 | 0,5 |
| Parfum | 0,4 | 0,4 | 0,4 | 0,4 | 0,4 |
| Eau qsp | 100 | 100 | 100 | 100 | 100 |
| Rapport pondéral de la quantité d'alcool(s) gras sur la quantité de mono-ou polyglycoside(s) | 11,5 | 7,6 | 9 | 9 | 9 |

| | | | | | |
|---|---|---|---|---|---|
| ⁽¹⁾ : selon l'exemple 2 de la demande WO 2008/155059. ⁽²⁾ : vendu sous la dénomination commerciale Vegelight 1214 par la société Byosynthis ⁽³⁾ : vendu sous la dénomination commerciale Lanette O OR par la société COGNIS ⁽⁴⁾ : vendu sous la dénomination commerciale Lanette 16 par la société COGNIS ⁽⁵⁾ : vendu sous la dénomination commerciale Montanov 68 par la société SEPPIC ⁽⁶⁾ : vendu sous la dénomination commerciale Montanov 82 par la société SEPPIC ⁽⁷⁾ : vendu sous la dénomination commerciale Tensolive par la société Soliance à 30% de matière active ⁽⁸⁾ : extrait d'orange biologique EBORAN 7050 de DIANA NATURAL | | | | | |

Ces compositions ont été testées sur têtes. On a appliqué chacune des compositions sur les cheveux et rincé ensuite.

On a observé un excellent lissage et une excellente souplesse à l'application et au rinçage.

On a également obtenu des cheveux humides plus faciles à démêler, plus légers et plus toniques, et après séchage, des cheveux séchés plus souples et plus lisses au toucher.

## Revendications

1. Composition cosmétique comprenant dans un milieu cosmétiquement acceptable :
- un ou plusieurs alcanes linéaires volatils,
- un ou plusieurs tensioactifs amphotères ou zwittérioniques,
- un ou plusieurs alcools gras en C₈₋₃₀, et
- un ou plusieurs mono- ou polyglycosides,
en un rapport pondéral alcool(s) gras/[mono- et/ou polyglycoside(s)] strictement supérieur à 2,5.

2. Composition cosmétique selon la revendication 1, **caractérisée en ce que** l'alcane linéaire volatil est un alcane linéaire comportant de 7 à 15 atomes de carbone, de préférence de 8 à 14 atomes de carbone.

3. Composition cosmétique selon la revendication2, **caractérisée en ce que** l'alcane linéaire volatil est choisi parmi le n-nonane, le n-undécane, le n-dodécane, le n-tridécane, le n-tétradécane, et leurs mélanges.

4. Composition cosmétique selon l'une quelconque des revendications précédentes, **caractérisée en ce que** l'alcane linéaire volatil est d'origine végétale.

5. Composition cosmétique selon l'une quelconque des revendications précédentes, **caractérisée en ce que** le ou les alcanes linéaires volatils sont présents en une teneur allant de 0,01 % à 30 % en poids, en particulier de 0,1 % à 20% en poids, et plus particulièrement de 0,1 % à 15% en poids par rapport au poids total de la composition.

6. Composition cosmétique selon l'une quelconque des revendications précédentes, **caractérisée en ce que** les alcools gras en C₈₋₃₀ sont choisis parmi les alcools laurylique, myristylique, cétylique, stéarylique, cétéarylique, oléique, arachidylique et béhénylique, l'hexyldécanol, le 2-octyldodécanol, et leurs mélanges.

7. Composition cosmétique selon l'une quelconque des revendications précédentes, **caractérisée en ce que** le ou les alcools gras en C₈₋₃₀ sont présents en une teneur allant de 0,5 à 20 % en poids, de préférence de 1 à 15% en poids, et plus particulièrement de 2 à 12% en poids par rapport au poids total de la composition.

8. Composition cosmétique selon l'une quelconque des revendications précédentes, **caractérisée en ce que** le mono- ou polyglycoside répond à la formule (I) :
R₁O-(R₂O)ₜ(G)ᵥ (I)
dans laquelle :
- R₁ représente un groupe alkyle et/ou alcényle, linéaire ou ramifié, comportant environ de 8 à 24 atomes de carbone, un groupe alkylphényle dont le groupe alkyle linéaire ou ramifié comporte de 8 à 24 atomes de carbone,
- R₂ représente un groupe alkylène comportant environ de 2 à 4 atomes de carbone,
- G représente un motif sucre comportant de 5 à 6 atomes de carbone,
- t désigne une valeur allant de 0 à 10, et
- v désigne une valeur allant de 1 à 15.

9. Composition cosmétique selon la revendication8, **caractérisée en ce que** le mono- ou polyglycoside est choisi parmi le caprylylglucoside, le décylglucoside, le laurylglucoside, le cétéarylglucoside, le cocoglucoside et leurs mélanges.

10. Composition cosmétique selon l'une quelconque des revendications précédentes, **caractérisée en ce que** le ou les mono- ou polyglycosides sont présents en une teneur allant de 0,1 à 20 % en poids, de préférence de 0,2 à 10% en poids, et plus particulièrement de 0,2 à 5% en poids par rapport au poids total de la composition.

11. Composition cosmétique selon l'une quelconque des revendications précédentes, **caractérisée en ce que** le rapport alcool(s) gras/[mono- et/ou polyglycoside(s)] va de de 2,6 à 50, mieux encore de 3 à 40, et encore mieux de 3 à 25.

12. Composition cosmétique selon l'une quelconque des revendications précédentes, **caractérisée en ce que** le ou les tensioactifs amphotères ou zwittérioniques sont choisis parmi les (alkyl en C₈₋₃₀)bétaïnes, les (alkyl en C₈₋₃₀)amido(alkyl en C₂₋₈)bétaïnes et leurs mélanges.

13. Composition cosmétique selon l'une quelconque des revendications précédentes, **caractérisée en ce qu'**elle comprend un ou plusieurs composés naturels ou d'origine naturelle, choisis parmi les actifs et extraits naturels, des huiles essentielles et des extraits de plantes, des huiles et beurres d'origine végétale.

14. Utilisation de la composition cosmétique selon l'une quelconque des revendications précédentes, pour le traitement des matières kératiniques, de préférence des cheveux.

15. Procédé de traitement des matières kératiniques, de préférence des fibres kératiniques, et mieux encore des cheveux, comprenant l'application de la composition cosmétique selon l'une quelconque des revendications 1 à 13 sur lesdites matières kératiniques.

## Patentansprüche

1. Kosmetische Zusammensetzung, die in einem kosmetisch unbedenklichen Medium Folgendes umfasst:
- ein oder mehrere flüchtige lineare Alkane,
- ein oder mehrere amphotere oder zwitterionische Tenside,
- einen oder mehrere C₈₋₃₀-Fettalkohole und
- ein oder mehrere Mono- oder Polyglykoside in einem Gewichtsverhältnis von Fettalkohol(en) zu Mono- oder Polyglykosid(en), das streng größer als 2,5 ist.

2. Kosmetische Zusammensetzung nach Anspruch 1, **dadurch gekennzeichnet, dass** es sich bei dem flüchtigen linearen n-Alkan um ein lineares Alkan mit 7 bis 15 Kohlenstoffatomen und vorzugsweise 8 bis 14 Kohlenstoffatomen handelt.

3. Kosmetische Zusammensetzung nach Anspruch 2, **dadurch gekennzeichnet, dass** das flüchtige lineare Alkan aus n-Nonan, n-Undecan, n-Dodecan, n-Tridecan, n-Tetradecan und Mischungen davon ausgewählt ist.

4. Kosmetische Zusammensetzung nach einem der vorhergehenden Ansprüche, **dadurch gekennzeichnet, dass** das flüchtige lineare Alkan pflanzlichen Ursprungs ist.

5. Kosmetische Zusammensetzung nach einem der vorhergehenden Ansprüche, **dadurch gekennzeichnet, dass** das oder die flüchtigen linearen n-Alkane in einem Gehalt im Bereich von 0,01 bis 30 Gew.-%, insbesondere 0,1 bis 20 Gew.-% und spezieller 0,1 bis 15 Gew.-%, bezogen auf das Gesamtgewicht der Zusammensetzung, vorliegen.

6. Kosmetische Zusammensetzung nach einem der vorhergehenden Ansprüche, **dadurch gekennzeichnet, dass** die C₈₋₃₀-Fettalkohole aus Laurylalkohol, Myristylalkohol, Cetylalkohol, Stearylalkohol, Cetearylalkohol, Oleylalkohol, Arachidylalkohol und Behenylalkohol, Hexyldecanol, 2-Octyldodecanol und Mischungen davon ausgewählt sind.

7. Kosmetische Zusammensetzung nach einem der vorhergehenden Ansprüche, **dadurch gekennzeichnet, dass** der oder die C₈₋₃₀-Fettalkohole in einem Gehalt im Bereich von 0,5 bis 20 Gew.-%, vorzugsweise 1 bis 15 Gew.-% und spezieller 2 bis 12 Gew.-%, bezogen auf das Gesamtgewicht der Zusammensetzung, vorliegen.

8. Kosmetische Zusammensetzung nach einem der vorhergehenden Ansprüche, **dadurch gekennzeichnet, dass** das Mono- oder Polyglykosid der Formel (I):
R₁O-(R₂O)ₜ(G)ᵥ (I)
entspricht, in der:
- R₁ für eine lineare oder verzweigte Alkyl- und/oder Alkenylgruppe mit ungefähr 8 bis 24 Kohlenstoffatomen oder eine Alkylphenylgruppe, deren lineare oder verzweigte Alkylgruppe 8 bis 24 Kohlenstoffatome aufweist, steht,
- R₂ für eine Alkylengruppe mit ungefähr 2 bis 4 Kohlenstoffatomen steht,
- G für eine Zuckereinheit mit 5 bis 6 Kohlenstoffatomen steht,
- t für einen Wert im Bereich von 0 bis 10 steht und
- v für einen Wert im Bereich von 1 bis 15 steht.

9. Kosmetische Zusammensetzung nach Anspruch 8, **dadurch gekennzeichnet, dass** das Mono- oder Polyglykosid aus Caprylylglucosid, Decylglucosid, Laurylglucosid, Cetearylglucosid, Cocoglucosid und Mischungen davon ausgewählt ist.

10. Kosmetische Zusammensetzung nach einem der vorhergehenden Ansprüche, **dadurch gekennzeichnet, dass** das oder die Mono- oder Polyglykoside in einem Gehalt im Bereich von 0,1 bis 20 Gew.-%, vorzugsweise 0,2 bis 10 Gew.-% und speziell 0,2 bis 5 Gew.-%, bezogen das Gesamtgewicht der Zusammensetzung, vorliegen.

11. Kosmetische Zusammensetzung nach einem der vorhergehenden Ansprüche, **dadurch gekennzeichnet, dass** das Gewichtsverhältnis von Fettalkohol(en) zu Mono- oder Polyglykosid(en) 2,6 bis 50, noch besser 3 bis 40 und noch besser 3 bis 25 beträgt.

12. Kosmetische Zusammensetzung nach einem der vorhergehenden Ansprüche, **dadurch gekennzeichnet, dass** das oder die amphoteren oder zwitterionischen Tenside aus (C₈₋₃₀-Alkyl) betainen, (C₈₋₃₀-Alkyl) - amido (C₂₋₈-Alkyl) betainen und Mischungen davon ausgewählt sind.

13. Kosmetische Zusammensetzung nach einem der vorhergehenden Ansprüche, **dadurch gekennzeichnet, dass** sie eine oder mehrere natürliche Verbindungen oder Verbindungen natürlichen Ursprung, die aus natürlichen Wirkstoffen und Extrakten, ätherischen Ölen und Pflanzenextrakten und Ölen und Buttern pflanzlichen Ursprungs ausgewählt sind.

14. Verwendung der kosmetischen Zusammensetzung nach einem der vorhergehenden Ansprüche zur Behandlung von Keratinmaterialien, vorzugsweise dem Haar.

15. Verfahren zur Behandlung von Keratinmaterialien, vorzugsweise Keratinfasern und noch besser dem Haar, bei dem man die kosmetische Zusammensetzung nach einem der Ansprüche 1 bis 13 auf die Keratinmaterialien aufbringt.

## Claims

1. Cosmetic composition comprising, in a cosmetically acceptable medium:
- one or more volatile linear alkanes,
- one or more amphoteric or zwitterionic surfactants,
- one or more C₈₋₃₀ fatty alcohols, and
- one or more mono- or polyglycosides,
in a fatty alcohol (s) / [mono- and/or polyglycoside(s)] weight ratio strictly greater than 2.5.

2. Cosmetic composition according to Claim 1, **characterized in that** the volatile linear alkane is a linear alkane comprising from 7 to 15 carbon atoms, preferably from 8 to 14 carbon atoms.

3. Cosmetic composition according to Claim 2, **characterized in that** the volatile linear alkane is chosen from n-nonane, n-undecane, n-dodecane, n-tridecane and n-tetradecane, and mixtures thereof.

4. Cosmetic composition according to any one of the preceding claims, **characterized in that** the volatile linear alkane is of plant origin.

5. Cosmetic composition according to any one of the preceding claims, **characterized in that** the volatile linear alkane(s) are present in a content ranging from 0.01% to 30% by weight, in particular from 0.1% to 20% by weight and more particularly from 0.1% to 15% by weight relative to the total weight of the composition.

6. Cosmetic composition according to any one of the preceding claims, **characterized in that** the C₈₋₃₀ fatty alcohols are chosen from lauryl alcohol, myristyl alcohol, cetyl alcohol, stearyl alcohol, cetearyl alcohol, oleyl alcohol, arachidyl alcohol, behenyl alcohol, hexyldecanol and 2-octyldodecanol, and mixtures thereof.

7. Cosmetic composition according to any one of the preceding claims, **characterized in that** the C₈₋₃₀ fatty alcohol(s) are present in a content ranging from 0.5% to 20% by weight, preferably from 1% to 15% by weight and more particularly from 2% to 12% by weight relative to the total weight of the composition.

8. Cosmetic composition according to any one of the preceding claims, **characterized in that** the mono- or polyglycoside corresponds to formula (I):
R₁O-(R₂O)ₜ(G)ᵥ (I)
in which:
- R₁ represents a linear or branched, alkyl and/or alkenyl group comprising from about 8 to 24 carbon atoms, an alkylphenyl group in which the linear or branched alkyl group comprises from 8 to 24 carbon atoms,
- R₂ represents an alkylene group comprising from about 2 to 4 carbon atoms,
- G represents a sugar unit comprising from 5 to 6 carbon atoms,
- t denotes a value ranging from 0 to 10, and
- v denotes a value ranging from 1 to 15.

9. Cosmetic composition according to Claim 8, **characterized in that** the mono- or polyglycoside is chosen from caprylyl glucoside, decyl glucoside, lauryl glucoside, cetearyl glucoside and cocoyl glucoside, and mixtures thereof.

10. Cosmetic composition according to any one of the preceding claims, **characterized in that** the mono- or polyglycoside(s) are present in a content ranging from 0.1% to 20% by weight, preferably from 0.2% to 10% by weight and more particularly from 0.2% to 5% by weight relative to the total weight of the composition.

11. Cosmetic composition according to any one of the preceding claims, **characterized in that** the fatty alcohol(s)/[mono- and/or polyglycoside(s)] ratio ranges from 2.6 to 50, better still from 3 to 40 and even better still from 3 to 25.

12. Cosmetic composition according to any one of the preceding claims, **characterized in that** the amphoteric or zwitterionic surfactant (s) are chosen from (C₈₋₃₀ alkyl) betaines and (C₈₋₃₀ alkyl) amido (C₂₋₈ alkyl) betaines, and mixtures thereof.

13. Cosmetic composition according to any one of the preceding claims, **characterized in that** it comprises one or more natural compounds or compounds of natural origin, chosen from natural active agents and extracts, essential oils and plant extracts, and oils and butters of plant origin.

14. Use of the cosmetic composition according to any one of the preceding claims, for treating keratin materials, preferably the hair.

15. Process for treating keratin materials, preferably keratin fibres, and better still the hair, comprising the application of the cosmetic composition according to any one of Claims 1 to 13 to said keratin materials.
